# EUROPEAN PATENT SPECIFICATION

(11) **EP 2 401 375 B1**
(45) Date of publication and mention of the grant of the patent: **23.08.2017**
(21) Application number: 10704945.4
(22) Date of filing: 24.02.2010
(51) Int. Cl.: C12N 15/11, C12N 15/113

(54) **IMPROVED DESIGN OF SMALL-INTERFERING RNA**
VERBESSERTE KONSTRUKTION VON SMALL-INTERFERING-RNA
CONCEPTION AMÉLIORÉE DE PETITS ARN INTERFÉRANTS

(30) Priority: 24.02.2009 EP 09153487
(43) Date of publication of application: 04.01.2012
(73) Proprietor: RiboxX GmbH, 01445 Radebeul (DE)
(72) Inventor: ROHAYEM, Jacques, 01277 Dresden (DE)
(74) Representative: Habermann, Hruschka & Schnabel
(86) International application number: PCT/EP2010/052348
(87) International publication number: WO 2010/097414

(56) References cited:
- WO-A2-2005/014782
- WO-A2-2007/012329
- US-A1- 2005 096 284

## Description

The present invention relates to small-interfering RNA molecules as defined in claim 1, displaying an increased thermodynamic stability at the 3' end of the antisense strand (guide strand) and the 5' end of the sense strand (passenger strand), respectively, in comparison to the base pairing in the seed region. The siRNAs of the present invention display an increased knock-down activity against targeted genes and show an improved resistance to RNAses, in particular serum RNAses. The present invention also relates to a pharmaceutical compositions containing the siRNA molecules.

RNA interference (RNAi) mediated by small double-stranded RNA (dsRNA) molecules is a well-established technique for gene silencing *in vitro* and *in vivo* (for a recent review see, e.g. Kim et al. (2007) Nature Reviews Genetics 8, 173-184, and the references cited therein).

siRNAs target complementary RNAs (in particular mRNA and viral RNA such as viral genomic or subgenomic RNA) by transactive-cleavage and degradation leading to post-transcriptional gene silencing (PTGS).

EP 1 407 044 B1 describes isolated dsRNA molecules in which each RNA strand has a length of from 19 to 23 nucleotides and wherein at least one strand has a 3'-overhang from 1 to 3 nucleotides, the RNA molecule being capable of target-specific RNA interference.

Other siRNA molecules suggested are corresponding molecules of 19 to 23 base pairs wherein the double-stranded RNA is blunt-ended on both sides.

EP 1 486 564 A1 describes a method for the specific selection of dsRNA molecules capable of RNA interference having improved efficiency through increased serum stability, wherein the sequences of the single strands of the dsRNA are selected such that at each end of the dsRNA the last complementary nucleotide pair is G-C or at least two of the last four complementary nucleotide pairs are G-C pairs wherein the dsRNA has an overhang of 1 to 4 unpaired nucleotides at the first end and has no overhang at the second end, wherein the single-stranded overhang is characterised in that the unpaired nucleotide directly next to the last complementary nucleotide pair contains a purine base. All specific dsRNA molecules selected by the method described in EP 1 486 564 A1 contain a 3' overhang at the guide strand.

US 2005/0096284 A1 discloses various prior art siRNAs for RNA interference-mediated treatment of polyglutamine (polyQ) repeat expension diseases.

WO 2005/014782 A2 discloses a method for the targeted selection of dsRNAs consisting of two single strands that exhibit increased effectiveness in inhibiting the expression of a target gene by means of RNA interference, wherein at least one end of the dsRNA comprises a nucleotide overhang of 1 to 4 unpaired nucleotides in length; wherein the unpaired nucleotide adjacent to the terminal nucleotide pair comprises a purine base; and wherein the terminal nucleotide pair on both ends of the dsRNA is a G-C pair, or at least two of the last four consecutive terminal nucleotide pairs are G-C pairs.

The technical problem underlying the present invention is to provide siRNA molecules displaying improved gene silencing activity.

The solution to the above technical problem is provided by an isolated double-stranded RNA molecule capable of target-specific RNA interference as defined in claim 1, which has an increased thermodynamic stability at the 5' end of the passenger strand and the 3' end of the guide strand, respectively, in comparison to the base pairing in the seed region of the siRNA molecule. The design of the siRNA molecules of the present invention leads to a remarkably increased gene silencing activity by (i) improving knock-down efficiency for the targeted gene and (ii) increasing the resistance to RNAses.

Thus, the present invention is specifically directed to an isolated dsRNA molecule capable of target-specific RNA interference, having 3 to 10 G/C base pairs added to the target sequence at the 5' end of the passenger strand and the 3' end of the guide strand such that said RNA molecule has an increased thermodynamic stability at the 5' end of the passenger strand and the 3' end of the guide strand, respectively, in comparison to the base paring of the first 10 nucleotides at the 5' end of the guide strand and wherein 3 to 10 G nucleotides are added to the 5' end of the target sequence at the passenger strand and 3 to 10 C nucleotides are added to the 3' end of the target sequence at the guide strand, wherein the 3' end of the passenger strand has no overhang or has an overhang of 1 to 3 nucleotides, wherein the RNA molecule has a length of 19 to 22 nucleotides excluding the G/C base pair sequences at the 5' end of the passenger strand and the 3' end of the guide strand, respectively, and excluding the optional overhang at the 3' end of the passenger strand, if present. The RNA molecule of the present invention has 3 to 10, i.e. 3, 4, 5, 6, 7, 8, 9 or 10 G/C base pairs added to the target sequence at the 5' end of the passenger strand and the 3' end of the guide strand, respectively. The RNA molecule of the invention is characterized in that the passenger strand has a sequence of 3 to 10, i.e. 3, 4, 5, 6, 7, 8, 9 or 10 G nucleotides added to the target sequence at the 5' end and the guide strand has a sequence of 3 to 10, i.e. 3, 4, 5, 6, 7, 8, 9 or 10 C nucleotides added to the target sequence at the 3' end. Most preferred, the RNA molecule of the present invention has a sequence of 5 G nucleotides added to the target sequence at the 5' end of the passenger strand and a sequence of 5 C nucleotides added to the target sequence at the 3' end of the guide strand.

The term "isolated double-stranded RNA molecule capable of target-specific RNA interference" according to the present invention is therefore directed to a double-stranded ribonucleotide in which one strand ("sense" or "passenger" strand) reflects the nucleotide sequence of a target RNA (e.g. mRNA of a targeted gene or viral RNA such as viral genomic or subgenomic RNA) and a second strand capable of forming a RNA duplex with the sense or passenger strand (the so-called "antisense" or "guide" strand), which displays an essentially complementary sequence to the "sense" or "passenger" strand and the targeted RNA, respectively. During the mechanism of post-translational gene silencing using siRNA molecules, only the antisense or guide strand is incorporated into the RISC complex (it is also possible that the passenger strand is loaded into the RISC complex, however, in this case the target RNA is not recognised and no RNA interference is initiated). The first ten nucleotides at the 5' end of the guide strand represent the so-called "seed" region which can be regarded as the core region required for recognition of the mRNA in the RISC complex. Therefore, it is preferred that the sequence within the seed region of the guide strand reflects the sequence of the targeted mRNA to 100 %.

dsRNA molecules of the present invention may contain a 3'-overhang of 1, 2 or 3 nucleotides at the passenger strand. Thus, on the opposite end (i.e. the end formed by the 5' end of the passenger strand and the 3' end of the guide strand) the dsRNA molecule of the invention is blunt ended (i.e. no overhang is present at this end). Furthermore, the siRNA molecules of the present invention have a length of from 19 to 22, particularly preferred 21 nucleotides excluding the G/C base pair sequences at the 5' end of the passenger strand and the 3' end of the guide strand, respectively, and excluding the optional overhang at the 3' end of the passenger strand, if present.

The stability of the double-stranded RNA molecules of the present invention may be increased by one or more of the following measures:
If present, a 3'-overhang at the passenger strand may be stabilized by the selection of a purine nucleotide, in particular adenosine or guanosine nucleotides. It is further possible to substitute pyrimidine nucleotides at the 3'-overhang by modified analogues, for example by substituting uridine nucleotides at the 3'-overhang by 2'-deoxythymidine. Furthermore, the absence of a 2'-hydroxyl group increases the nuclease resistance of a 3'-overhang, in particular in tissue culture media.

Furthermore, the RNA molecule may also contain at least one modified nucleotide analogue, in particular within the dsRNA forming region.

The chemical modification of the nucleotide analogue in comparison to the natural occurring nucleotide may be at the ribose, phosphate and/or base moiety. With respect to molecules having an increased stability, especially with respect to RNA degrading enzymes, modifications at the backbone, i. e. the ribose and/or phosphate moieties, are especially preferred.

Preferred examples of ribose-modified ribonucleotides are analogues wherein the 2'-OH group is replaced by a group selected from H, OR, R, halo, SH, SR, NH₂, NHR, NR₂ or CN with R being C₁-C₆ alkyl, alkenyl or alkynyl and halo being F, Cl, Br or I. It is clear for the person skilled in the art that the term "modified ribonucleotide" also includes 2'-deoxyderivatives which may at several instances also be termed "deoxynucleotides". Examples of 2'-O-modified analogues such as 2'-O-methyl analogues may also be included in the seed region, e.g. in the 2 position of the guide strand (counted from its 5' end) in order to minimise off-target effects.

As mentioned before, the at least one modified ribonucleotide may be selected from analogues having a chemical modification at the base moiety. Examples of such analogues include, but are not limited to, 5-aminoallyl-uridine, 6-aza-uridine, 8-aza-adenosine, 5-bromo-uridine, 7-deaza-adenosine, 7-deaza-guanosine, N⁶-methyl-adenosine, 5-methyl-cytidine, pseudo-uridine and 4-thio-uridine.

Examples of backbone-modified ribonucleotides wherein the phosphoester group between adjacent ribonucleotides is modified are phosphothioate groups.

If present, the 3'-overhang of the passenger strand can also be labelled for detection in analytical, in particular diagnostic, applications. The "label" can be any chemical entity which enables the detection of the siRNA in question via physical, chemical and/or biological means. Examples of typical labels linked to or integrated into one or more of the nucleotides of the 3'-overhang are radioactive labels, chromophores and fluorophores (e.g. fluorescein, TAM etc.).

The sequence identity of the passenger strand within its portion excluding the G/C base pairs and, optionally, the 3'-overhang, is preferably at least 70 % to the desired target molecule (i. e. the targeted RNA). More preferably, the identity is at least 85 % and most preferably 100 %. In turn, this means that the complementarity of the sequence in the guide strand is at least 70 %, more preferably 85 %, most preferred 100 % to the desired target RNA. In particular, it is most preferred that the above-defined seed region of the guide strand has 100 % complementarity to the target RNA.

Exemplary, and particular preferred, siRNA molecules of the present invention are the sequence pairs (passenger strand/guide strand) of SEQ ID NO: 5/SEQ ID NO: 6, SEQ ID NO: 17/ SEQ ID NO: 18, SEQ ID NO: 19/ SEQ ID NO: 20, SEQ ID NO: 21/ SEQ ID NO: 22, SEQ ID NO: 23/ SEQ ID NO: 24 and SEQ ID NO: 25/ SEQ ID NO: 26.

Also disclosed is a method for the production of the above-defined isolated dsRNA molecule comprising the steps of:
(a) preparing a passenger strand having a sequence of at least 3 G and/or C nucleotides at its 5' end, optionally having an overhang of 1 to 5 nucleotides at its 3' end (i.e. the passenger strand may be 1 to 5 nucleotides longer than the guide strand), and a guide strand having a sequence of at least 3 G and/or C nucleotides at its 3' end wherein the sequence of the at least 3 G and/or C nucleotides at the 5' end of the passenger strand and the sequence of the at least 3 G and/or C nucleotides at the 3' end of the guide strand are complementary (i.e. the sequences of the passenger and guide strand are complementary, excluding the optional overhang of 1 to 5 nucleotides at the 3' end of the passenger strand);
(b) combining the passenger strand and the guide strand under conditions that a double-stranded RNA molecule, optionally having an overhang of 1 to 5 nucleotides at the 3' end of the passenger strand, is formed.

The strands of the double-stranded RNA molecule of the present invention may be prepared by chemical synthesis or enzymatically. When the strands of the double-stranded RNA molecule of the present invention are prepared by chemical synthesis, the ultimate nucleotide at the 5'-terminus of the passenger strand bears a monophosphate at the 5'-Position of the ribose. When the strands of the double-stranded RNA molecule of the present invention are prepared by enzymatic synthesis, the ultimate nucleotide at the 5'-terminus of the passenger strand bears a triphosphate at the 5'-Position of the ribose. The present invention is therefore also directed to dsRNA molecules having either a monophosphate group or a triphosphate group at the 5' position of the ribose of the 5' terminal nucleotide of the passenger strand.

Chemical synthesis methods may include, for example, the phosphotriester method described by Narang et al. (1979) Methods in Enzymology 68: 90, the phosphodiester method disclosed by Braun et al. (1979) Methods in Enzymology 68: 109, the diethylphosphoamidate method disclosed in Beaucage et al. (1981) Tetrahydron Letters 22: 1859, and the solid support method disclosed in US-A-4,458,066.

Enzymatic methods for the preparation of the double-stranded RNA molecules of the present invention preferably make use of RNA-dependent RNA-polymerases (RdRPs). Examples of RdRPs are corresponding enzymes from viruses such as caliciviruses (e.g. norovirus, sapovirus, vesivirus, lagovirus) and Hepatitis C virus etc. With respect to caliciviruses it is referred to WO-A-2007/012329. Alternatively, single RNA strands as defined in step (a) of the method for the preparation of the siRNA molecule of the invention can be prepared by DNA-dependent RNA polymerases from synthetic DNA templates or from DNA plasmids which may be prepared from recombinant host cells capable of propagating said plasmids. DNA-dependent RNA polymerases useful in this respect are preferably RNA polymerases from phages such as T7, T3 or SP6 RNA polymerase (see, e.g. Milligan et al. (1989) Methods Enzymol. 180, 51-62).

In particular, for the production of double-stranded RNA molecules according to the present invention on a larger scale, it is preferred that a small amount of the desired dsRNA molecules is first prepared by a chemical synthesis method (examples are mentioned above) followed by an enzymatic amplification of the dsRNA, preferably using a RdRP of a calicivirus as disclosed in WO-A-2007/012329. With respect to the enzymatic production of backbone-modified dsRNA molecules using RdRps, in particular RdRps from the family of *Caliciviridae*, it is referred to WO-A-2009/150156.

The isolated double-stranded RNA molecules of the present invention are particularly for use in target-specific RNA interference. Also disclosed is a method of target-specific RNA interference comprising the step of contacting a eukaryotic cell or organism with a double-stranded RNA molecule (or more than one double-stranded RNA molecule) as defined above wherein the cell or organism contains a target RNA (e.g. an mRNA expressed by said cell or organism, or viral RNA such as genomic or subgenomic viral RNA) having a sequence identity of at least 70 % to the passenger strand of the double-stranded RNA molecule. It is disclosed that preferred values of sequence identities between the passenger strand and the target mRNA are as defined above.

It is also disclosed that the double-stranded RNA molecule(s) is/are introduced into the cell or organism. Methods for introducing the siRNA of the present invention into a cell or organism are known to the skilled person. Protocols of methods for introducing nucleic acids, in particular siRNA molecules, into cells/organisms can be found, e.g. in the latest edition of Ausubel et al. (ed.) Current Protocols in Molecular Biology, John Wiley & Sons, New York, USA.

The double-stranded RNA molecules of the present invention are for use as a medicament and/or diagnostic tools as well as tools for experimental research in the field of basic and applied sciences.

For medical purposes the RNA molecule according to the present invention is preferably contained in a pharmaceutical composition in combination with at least one pharmaceutical carrier, excipient and/or diluent. The formulations for pharmaceutical compositions in the context of the present invention, and their routes of administration are known to the skilled person, and guidance may be found in the latest edition of Remington's Pharmaceutical Sciences (Mack Publishing Co., Easton, PA, USA).

An important pre-requisite for therapeutic use of RNA-inteferring molecules relates also to the resistance of the molecules to RNAses. RNAses are ubiquitary in living organisms and are especially abundant in serum and other body fluids. Degradation of RNA-intereferring-molecules by serum RNAses so far hampers the broad therapeutic application of these molecules, in particular for intra-venous administration. One approach to solve this problem has been a chemical modification of nucleotides in the sense and or antisense strand, usually the introduction of 2'-O-CH₃ or 3'-fluoro groups. Although increasing the RNAse resistance, such chemical modifications often lead to a decrease in the gene silencing efficiency.

The present invention allows through a new design of sense/antisense strands of the RNA-interfering molecules to provide siRNAs having an increased resistance to RNAses, even without having any chemical modification in the nucleotides, hence conserving the gene silencing efficiency.

Furthermore, the present invention is also directed to a diagnostic kit containing the dsRNA of the present invention in combination with at least one means for the detection of the dsRNA. The diagnostic kit preferably contains chemicals for the labelling (e.g. radioactively labelled entities, chromophores or fluorophores) and/or detection of a labelled dsRNA of the invention. The kit according to the present invention may also contain one or more pre-labelled dsRNA as defined herein (e.g. a dsRNA having a 3'-overhang labelled with a fluorescent group as exemplified above).

The siRNA molecules of the present invention are particularly useful in the treatment of diseases, including infectious diseases caused by an infectious agent such as a bacterium, virus or fungus, and tumours. With respect to siRNA molecules of the invention, preferably contained in a corresponding pharmaceutical composition, against viral diseases, it is preferred that the siRNA is directed against a virus, preferably selected from the group consisting of Caliciviridae, Orthomyxoviridae, Paramyxoviridae, Picornaviridae, Flaviviridae, Togaviridae, Hepaciviridae and Astroviridae. Preferred Caliciviridae to be combated with the siRNA molecules of the present invention include norovirus, sapovirus, lagovirus and vesivirus. The sequence of the siRNA of the present invention may therefore be chosen to decrease, preferably abolish the expression of a gene of an infectious agent or tumour.

Especially preferred siRNA molecules of the present invention are directed against Feline Calicivirus (FCV) and include the siRNA having a sense (= passenger) strand according to SEQ ID NO: 5 and an antisense (= guide) strand according to SEQ ID NO: 6. Further preferred siRNAs of the invention are directed against Human Rhinovirus Type 1B (HRV 1B) and include the sequence pairs (passenger/guide strand) of SEQ ID NO: 17/ SEQ ID NO: 18, SEQ ID NO: 19/ SEQ ID NO: 20, SEQ ID NO: 21/ SEQ ID NO: 22, SEQ ID NO: 23/ SEQ ID NO: 24 and SEQ ID NO: 25/ SEQ ID NO: 26.

There is also disclosed a method for the treatment of a disease, preferably a viral (examples are outlined above) or tumour disease, comprising administering an effective amount of the pharmaceutical composition of the invention to a preferably mammalian, in particular a human, patient in need of such treatment.

The present invention also relates to a eukaryotic cell or eukaryotic non-human organism comprising the double-stranded RNA molecule as defined in claim 1 or comprising a DNA coding therefor.

The figures show:

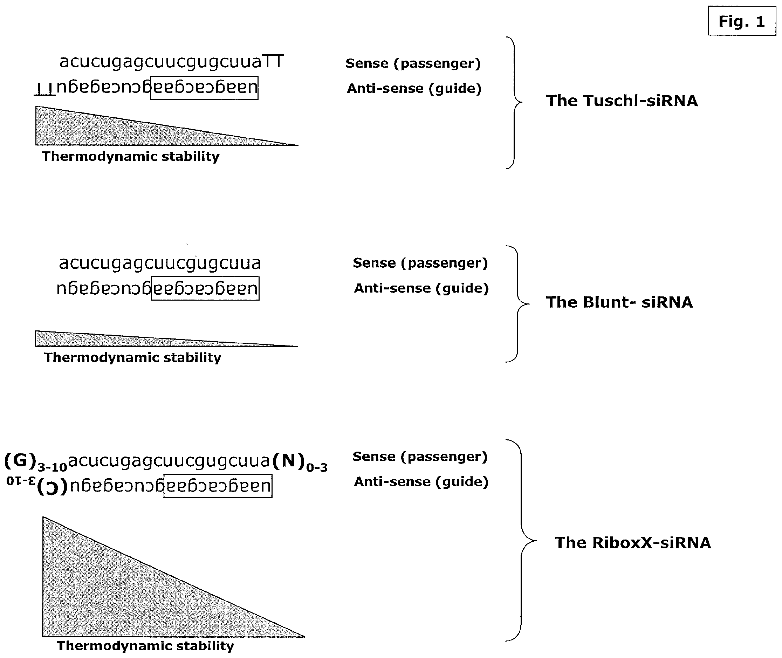

- Fig. 1: is a schematic overview of the design of a dsRNA of the present invention (in the following also referred to as "RiboxX-siRNA") in comparison to a prior art siRNA having a design disclosed in EP 1 407 044 B1 ("Tuschl-siRNA") and blunt-end siRNA ("Blunt-siRNA"). A heteropolymeric sequence of 19 nt is used as an example. The seed region is highlighted by a box. The Tuschl-siRNA is characterized by a length of 19-25 nt with 2 nucleotides overhang at the 3' end. The blunt-end siRNA does not exhibit any overhangs, and its length varies from 19-25 nt. The RiboxX-siRNA (preferably 18-26 nt) displays at the 3' end of the antisense strand (guide strand) and the 5' end of the sense strand (passenger strand) a G/C-Base pairing, preferably of 3 to 10 bp in length. At the 3' end of the passenger strand, there is possibly an overhang, preferentially of 1 to 5 nucleotides. The design of the siRNA of the present invention provides for a higher thermodynamic stability at the 3' end of the antisense strand (guide strand) and the 5'-end of the sense strand (passenger strand), in comparison to the base pairing in the seed region (highlighted by a box). This thermodynamic stability leads in turn to a more efficient processing of the RiboxX-siRNA through the RISC complex and subsequent loading of the antisense strand.

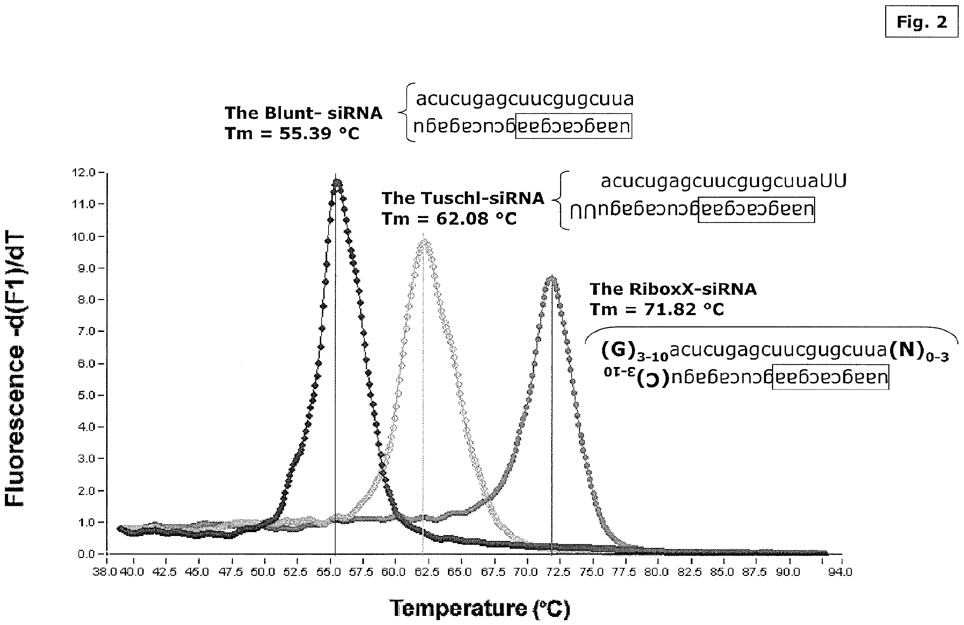

- Fig. 2: is a graphical representation of melting point experiments for comparing the thermodynamic stability of a siRNA according to the invention ("RiboxX-siRNA") with a siRNA having a design as described in EP 1 407 044 B1 ("Tuschl-siRNA") and a blunt-end siRNA ("Blunt-siRNA"). A heteropolymeric sequence of 19 nt is used as an example. The seed region is highlighted by a box. The thermodynamic stability is assessed through the melting point (Tm) of the double-stranded RNA (dsRNA). The Tm is determined after incubation of the dsRNA with SYBR-Green and measurement of the Tm with the LightCycler (Roche Diagnostics GmbH, Mannheim, Germany). SYBR-Green is an intercalating fluorescent dye that binds to the dsRNA, and not to the single-stranded (ss)RNA. Upon increase of temperature in the reaction, the Tm of the dsRNA-Hybrid is reached and the dsRNA melts, releasing the SYBR-Green, with a subsequent decrease in fluorescence which is measured by the LightCycler. The integration of the curve as a differential equation allows the accurate determination of the Tm as a peak, as indicated. VC, virus control.

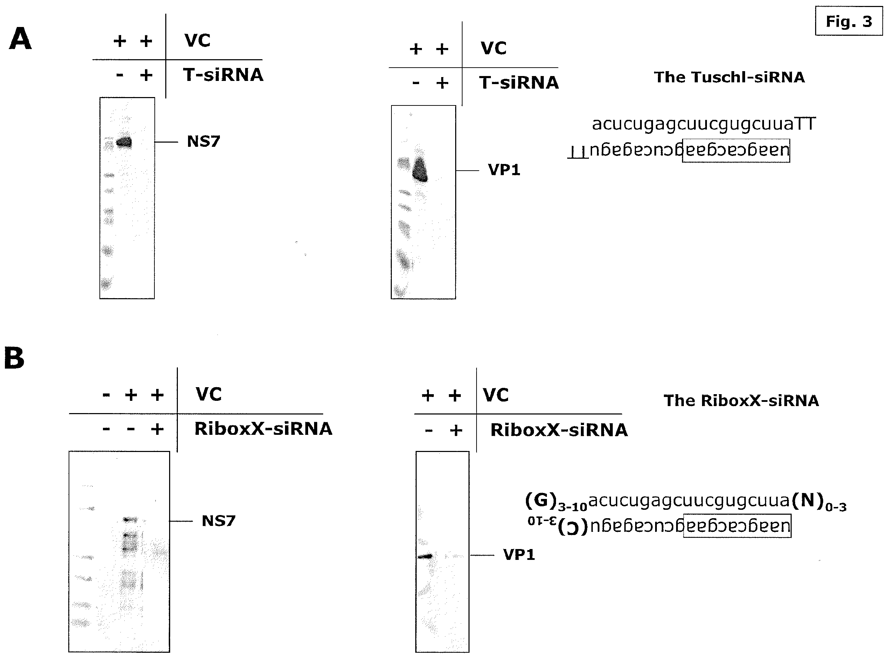

- Fig. 3: shows photographs of western-blot analyses for the qualitative assessment of the knock-down activity of a siRNA according to the invention ("RiboxX-siRNA") in comparison to a corresponding prior art siRNA having a design as suggested in EP 1 407 044 B1 ("Tuschl-siRNA"). A heteropolymeric sequence of 19 nt is used as an example. The seed region is highlighted by a box. The activity of the RiboxX-siRNA has been evaluated in a virus model of infection. In the first step, a cell monolayer (CRFK-Cells) has been treated with or without the siRNA 4 hours pre-infection. Then, a virus titre of the feline calicivirus has been used to infect the cell monolayer (CRFK-Cells). In the second step, the cellular and viral proteins were purified and used for western-blot analysis with specific antibodies directed against the NS7-Polymerase or the VP1-capsid gene of the feline calicivirus. (A), Western-blot analysis of the knock-down of the expression of the NS7-Polymerase gene and the VP1-Capsid gene of the feline calicivirus upon treatment with the Tuschl-siRNA (abbreviated as "T-siRNA"). (B) Western-blot analysis of the knock-down of the expression of the NS7-Polymerase gene and the VP1-Capsid gene of the feline calicivirus upon treatment with the RiboxX-siRNA.

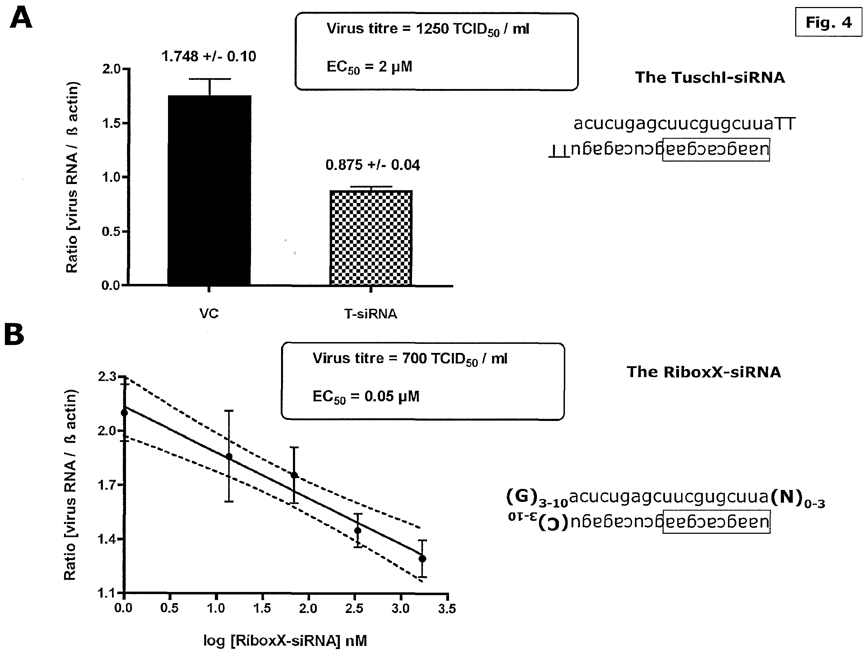

- Fig. 4: shows graphical representations of experimental results for the quantitative assessment of the knock-down activity of a siRNA according to the present invention ("RiboxX-siRNA") in comparison to a siRNA having a design as disclosed in EP 1 407 044 B1 ("Tuschl-siRNA"). A heteropolymeric sequence of 19 nt is used as an example. The seed region is highlighted by a box. The activity of the RiboxX-siRNA has been evaluated in a virus model of infection (feline calicivirus). In the first step, a cell monolayer (CRFK-Cells) has been treated with or without the siRNA 4 hours pre-infection. Then, a virus titre of the feline calicivirus has been used to infect the cell monolayer (CRFK-Cells), as indicated. In the second step, the cellular and viral RNA were extracted, purified and used for quantitative real-time PCR of the viral genome. The value of viral RNA has been normalized against a house-keeping gene, here β-actin. (A) Reduction of 50% of the viral genome expression evidenced by quantitative real-time PCR when the infected cell monolayers were treated with the siRNA according to EP 1 407 044 B1 ("Tuschl-siRNA"). The EC₅₀ (effective concentration that knocks-downs the expression of viral RNA in 50% of the experiments) was determined to be 2 µM. The mean values +/- SEM of 4 independent measurements are shown. (B) Dose-dependent reduction of the viral genome expression evidenced by quantitative real-time PCR when the infected cell monolayers were treated with the RiboxX-siRNA. The mean values +/- SEM of 4 independent measurements are shown. VC, Virus control. The tested siRNA according to the present invention displays an EC₅₀ value of only 0.05 µM, thus being 40-fold lower than the EC₅₀ value of a siRNA having a design according to the prior art (in particular EP 1 407 044 B1).

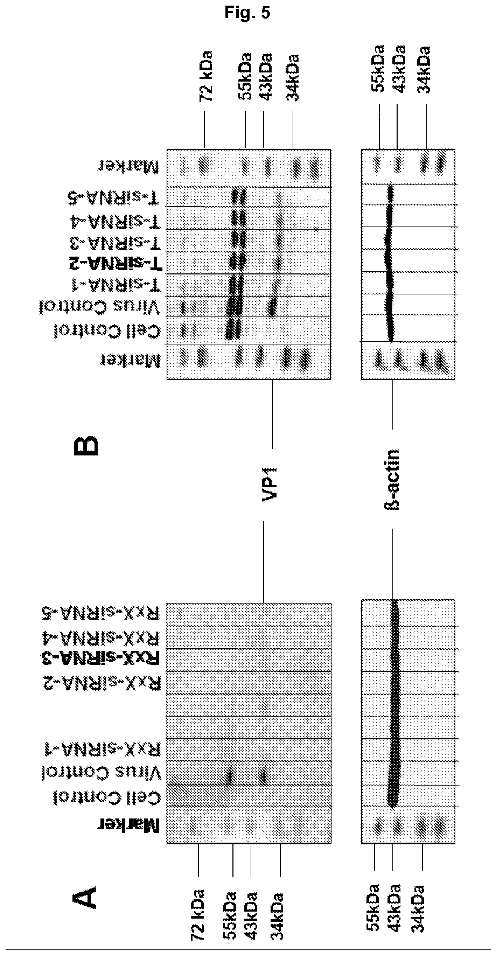

- Fig. 5: shows photographs of western-blot analyses for the assessment of the knock-down activity of further siRNAs according to the present invention ("RiboxX-siRNA") in comparison to a siRNA having a design as disclosed in EP 1 407 044 B1 ("Tuschl-siRNA"), using the same concentration of 20 nM for each of the siRNAs. Five different heteropolymeric sequences of 19 nt are used as examples (RxX-RNA-1 to -5 and T-RNA-1 to 5, respectively). The activity of the siRNA according to the present invention has been evaluated in a virus model of infection (human rhinovirus type 1B, HRV1B). (A) Western-blot analysis of the knock-down of the expression of the VP1-capsid gene upon treatment with 20 nM of the RiboxX-siRNA (RxX-siRNA-1 to RxX-siRNA-5). Expression of a cellular gene (β-actin) served as a control. (B), Western-blot analysis of the knock-down of the expression of the VP1-capsid gene of the HRV1B upon treatment with 20 nM of the prior art siRNA (T-siRNA-1 to T-siRNA-5). Expression of a cellular gene (β-actin) served as a control. Cell control relates to the cells non-infected with the virus, and virus control relates to the cell infected with the virus (Multiplicity of infection 0.05) but not treated with siRNA. Marker: molecular weight marker as indicated.

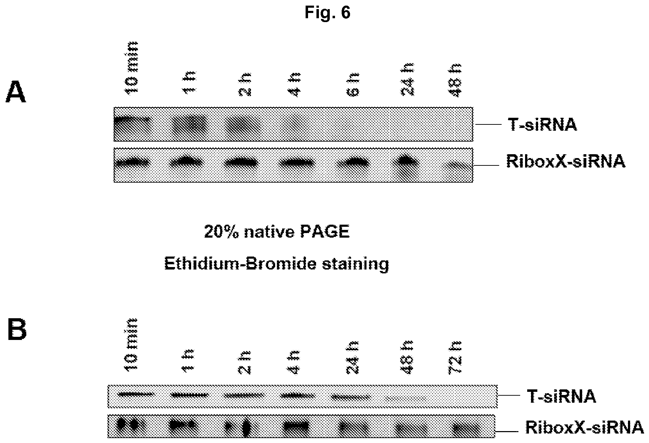

- Fig. 6: shows photographs of polyacrylamide gels after ethidium bromide staining for the assessment of the serum stability of siRNA according to the invention ("RiboxX-siRNA") in comparison to corresponding prior art siRNA having a design as suggested in EP 1 407 044 B1 ("Tuschl-siRNA"). In order to assess the resistance of siRNA to serum RNAses, siRNA displaying the design according to the invention (RX-siRNA) or the prior art design (T-siRNA) were incubated with fetal calf serum (panel A) or mouse serum (panel B) and withdrawn at the indicated time points and analyzed by PAGE.

The present invention is further illustrated by the following non-limiting examples.

### EXAMPLES

### Example 1: Sequence of dsRNA of the invention in comparison to prior art design

The sequences of the sense and the antisense strands of the siRNAs used in the following experiments (results see Figs. 2 to 4) were as follows:
"Tuschl-" or "T-siRNA" (comparative siRNA):
   - Sense (5'-3'-orientation): acucugagcuucgugcuuaTT (SEQ ID NO: 1)
   - Antisense (5'-3'-orientation): uaagcacgaagcucagaguTT (SEQ ID NO: 2)
"blunt-siRNA" (comparative siRNA):
   - Sense (5'-3'-orientation): acucugagcuucgugcuua (SEQ ID NO: 3)
   - Antisense (5'-3'-orientation): uaagcacgaagcucagagu (SEQ ID NO: 4)
"RiboxX-siRNA" (siRNA of the invention):
   - Sense (5'-3'-orientation): GGGGGacucugagcuucgugcuua (SEQ ID NO: 5)
   - Antisense (5'-3'-orientation): uaagcacgaagcucagaguCCCCC (SEQ ID NO: 6)

The above siRNAs target the 5'-UTR (untranslated region) of the viral genome of the Feline Calicivirus FCV/DD/2006/GE (GenBank acc. No. DQ424892), corresponding to positions 29 to 47 in the viral genome.

### Example 2: Increased thermodynamic stability of siRNA according to the invention in comparison to prior art siRNAs

For determination of the Tm, the respective sense and antisense RNA strands as outlined in Example 1 were annealed and the dsRNA incubated with SYBRGreen (Invitrogen). The annealing reaction with a total volume of 16 µl consisted of 4 µl of sense RNA (1 µg/µl) and 4 µl of antisense RNA (1 µg/µl), 3 µl of annealing buffer (Tris-Cl pH 7.8, NaCl 150 mM), and 5 µl RNAse-Dnase-free water. The reaction was incubated for 30 min at 65°C, then chilled on ice for 15 min.

Eight microliter of the respective annealed dsRNA was incubated in a total reaction volume of 25 µl with 2.5 µl SYBRGreen (invitrogen) and 14.5 µl RNAse-Dnase-free water. The reaction was then run on the LightCycler (ROCHE) and real-time measurement of the fluorescence intensity under F1 channel form 37°C till 95°C. The Fluorescence curve was then integrated as a differential equation of the fluorescence as a function of time (Fig. 2).

Fig. 2 shows that the RNA according to the invention ("RiboxX-siRNA") has an increased Tm (71.82 °C) compared to prior art siRNAs (Blunt-siRNA: 55.39 °C; Tuschl-siRNA: 62.08 °C).

### Example 3: siRNA according to the invention knocks down virus activity

A cell monolayer (CRFK-Cells, cultivated in a 6-Well-Plate and seeded at 300.000 cells per well) has been treated with or without the siRNAs as shown in Example 1 (T-siRNA and RiboxX-siRNA) for 4 hours pre-infection. Then, a virus titre of the feline calicivirus (750-1250 TCID₅₀/ml) has been used to infect the cell monolayer (CRFK-Cells). In the second step, the cellular and viral proteins were extracted and purified. Therefore, after removal of the supernatant from the transfected cells, monolayers were resolved by adding 600 µl of 2xPPP-Buffer. The cell lysates were collected in a QIAShredder (Qiagen) and centrifuged at 13,000 rpm for 2 minutes, followed by incubation with 600 µl aqua dest at 95°C for 5 min.

For western blot analysis, 80 µl of cell lysate were analyzed by SDS-PAGE and transferred onto Hybond-ECL nitrocellulose membranes. The membranes were incubated at 4°C for 16h with PBS / 0.1% Tween 20 and 5% skim milk powder with rabbit polyclonal antibody of interest (anti-NS7 or anti-VP1-capsid) as the primary antibody for 2h at room temperarture. After washing three times with PBS / 0.1 % Tween 20, the secondary antibody coupled with horseradish peroxidase was added. Detection was performed with the SuperSignal West Pico Chemiluminescent Substrate (Pierce) according to the manufacturer's instructions on a LAS-3000 Biolmager (FujiFilm).

### Example 4: siRNA of the invention is more efficient than prior art siRNA when applied in a virus model of infection

The antiviral activity of the T-siRNA and the RiboxX-siRNA of Example 1 were assessed quantitatively in the virus model of infection used in Example 3.

A cell monolayer (CRFK-Cells, cultivated in a 6-Well-Plate and seeded at 300.000 cells per well) has been treated with or without the T-siRNA or RiboxX-siRNA, respectively, for 4 hours pre-infection. Then, a virus titre of the feline calicivirus has been used to infect the cell monolayer (CRFK-Cells), as indicated in Fig. 4A and B, respectively. In the second step, the cellular and viral RNA were extracted using the RNAeasy Mini kit (Qiagen), purified and used for quantitative real-time PCR of the viral genome. The value of viral RNA has been normalized against a house-keeping gene, here β-actin. The quantitative real-time PCR consisted of a reverse transcription step, followed by a polymerase chain reaction. For reverse transcription, both the target RNA and the β actin mRNA were transcribed using an oligo(dT)₂₀. The reverse transcription reaction mix (20 µl) consisted of 5 µl RNA template, 4 µl of 5x Superscript-Buffer, 0.4 mM of each dATP, dCTP, dGTP and dTTP, 1µM of oligo(dT)₂₀, and 1µl of Superscript III (200 U / µl, Invitrogen), and RNAse-DNAse-free water to a final volume of 20 µl. Reverse transcription was carried out for 60 min at 50°C.

For amplification of the viral RNA, real-time PCR assays were run on Roche LightCycler 1.5 (Roche diagnostics). The reaction with a total volume of 20 µl consisted of 4.8 µl MgCl₂, 2 µl of each forward and reverse primer (5 pmol / µl), 2 µl TaqMan probe (2 pmol / µl) and 2 µl Roche HotStart reaction mix, and and RNAse-DNAse-free water to a final volume of 20 µl. Five microliters template were added to each reaction. The PCR cycling conditions were 10 minutes of pre-incubation at 95°C, annealing for 5 seconds at 61°C, and extension for 5 seconds at 72°C. At the end of the extension step, fluorescence signal was measured at 560 and 640 nm (F1/F2). To complete the run, capillaries were cooled down to 40°C for 2 minutes. The crossing-point values were determined for each sample. Additionally, a PCR specific for the house-keeping gene β-actin was performed as an internal control. Similarly, the crossing-point values for each sample were determined.

As is demonstrated by the EC₅₀ value (see Fig. 4), the siRNA of the invention displays a 40-fold higher efficiency in this experimental setting as compared to a siRNA having a design according to the prior art.

### Example 5: Sequence of further siRNAs of the invention in comparison to further prior art siRNAs

The sequences of the sense (= passenger) and the antisense (= guide) strands of the siRNAs used in the following experiments (results see Figs. 5 and 6) were as shown in the following table:
The corresponding nucleotides in the sequence of the human rhinovirus Type 1 B genome (GenBank Acc. No. D00239) for the passenger strand of the T-siRNA (excluding the last two UU at the 3'-terminus) and the passenger strand of the RxX-siRNA (excluding the first five GGGGG at the 5'-terminus of the passenger strand), respectively, is indicated.

| **Name** | **Passenger strand / Sense (5'-3)** | **Guide strand / Antisense (5'-3')** | **Position in the HRV 1B genome (GenBank Acc. No. D00239) relating to the passenger strand** |
|---|---|---|---|
| T-siRNA-1 | GCUUGUGGCUAUUCAGAUAUU (SEQ I D NO: 7) | UAUCUGAAUAGCCACAAGCUU (SEQ ID NO: 8) | 845-863 |
| T-siRNA-2 | UGAAAGUGAUGUGUAUUCAUU (SEQ ID NO: 9) | UGAAUACACAUCACUUUCAUU (SEQ ID NO: 10) | 4323-4351 |
| T-siRNA-3 | UUGGUCAGAUUCUAGGUAUUU (SEQ ID NO: 11) | AUACCUAGAAUCUGACCAAUU (SEQ ID NO: 12) | 5625-5643 |
| T-siRNA-4 | GCAAGUAGCAUAAAUGAUAUU (SEQ ID NO: 13) | UAUCAUUUAUGCUACUUGCUU (SEQ ID NO: 14) | 6245-6263 |
| T-siRNA-5 | ACUGCUGACAUGUUGUGUUUU (SEQ ID NO: 15) | AACACAACAUGUCAGCAGUUU (SEQ ID NO: 16) | 2234-2252 |
| Riboxx-siRNA-1 | GGGGGCUUGUGGCUAUUCAGAUA (SEQ ID NO: 17) | UAUCUGAAUAGCCACAAGCCCCC (SEQ ID NO: 18) | 845-863 |
| Riboxx-siRNA-2 | GGGGGUGAAAGUGAUGUGUAUUCA (SEQ ID NO: 19) | UGAAUACACAUCACUUUCACCCCC (SEQ ID NO: 20) | 4323-4351 |
| Riboxx-siRNA-3 | GGGGGUUGGUCAGAUUCUAGGUAU (SEQ ID NO: 21) | AUACCUAGAAUCUGACCAACCCCC (SEQ ID NO: 22) | 5625-5643 |
| Riboxx-siRNA-4 | GGGGGCAAGUAGCAUAAAUGAUA (SEM ID NO: 23) | UAUCAUUUAUGCUACUUGCCCCC (SEQ ID NO: 24) | 6245-6263 |
| Riboxx-siRNA-5 | GGGGGACUGCUGACAUGUUGUGUU (SEQ ID NO: 25) | AACACAACAUGUCAGCAGUCCCCC (SEQ ID NO: 26) | 2234-2252 |

### Example 6: Increased knock-down activity of Riboxx siRNAs 1 to 5 according to the invention compared to T-siRNAs 1 to 5 having prior art design

The siRNAs with the sequences according to Example 5 were assessed for gene knock-down efficiency in a virus model of infection using human rhinovirus type 1 B (HRV1 B).

A cell monolayer (HeLa-Cells) was treated with or without the respective siRNA (20 nM) 4 hours pre-infection. Then, a virus titre of HRV1B was used to infect the cell monolayer (HeLa-Cells). The cellular and viral proteins were purified and used for western-blot analysis with specific antibodies directed against a viral (VP1-capsid protein) or a cellular protein (β-actin). For western blot analysis, 80 µl of cell lysate were analyzed by SDS-PAGE and transferred onto Hybond-ECL nitrocellulose membranes. The membranes were incubated at 4°C for 16 h with PBS / 0.1 % Tween 20 and 5% skim milk powder with rabbit polyclonal antibody of interest (anti-β-actin or anti-VP1) as the primary antibody for 2 h at room temperarture. After washing three times with PBS / 0.1% Tween 20, the secondary antibody coupled with horseradish peroxidase was added. Detection was performed with the SuperSignal West Pico Chemiluminescent Substrate (Pierce) according to the manufacturer's instructions on a LAS-3000 Biolmager (FujiFilm).

As shown in Figs. 5A and B, the siRNAs according to the present invention (Riboxx-RNA 1 to 5) efficiently block expression of viral protein whereas siRNAs having prior art design do not efficiently knock down the expression of the viral protein, using the same concentration of 20 nM for each of the siRNA.

### Example 7: siRNAs according to the present invention show increased resistance against serum RNAses

In order to assess the resistance of siRNA to serum RNAses, siRNA displaying the design according to the invention (RxX-siRNA-3) or the prior art design (T-siRNA-3) were incubated with fetal calf serum or mouse serum and withdrawn at different time points and analyzed with PAGE. The reaction mixture (total volume of 50 µl) contained 3 µM siRNA in 80% fetal calf serum (FCS) and 20% DMEM (v/v). The reaction was incubated at 37°C and 900 rpm, and 5 µl of the reaction were withdrawn at 10 min, 1 h, 2 h, 4 h, 6 h, 24 h, and 48 h and subsequently analyzed by 20% native PAGE.

The siRNA having the prior art design (T-siRNA) is completely degraded 4 h after incubation in FCS, whereas the siRNA displaying the design according to the invention is still detected at 48 h post incubation with FCS (Fig. 6A). In the case of incubation with mouse serum, the siRNA having the prior art design (T-siRNA) is completely degraded 72 h after incubation in mouse serum, whereas the siRNA according to the present invention is still detected at 72 h post incubation with mouse serum (Fig. 6B).

## Claims

1. An isolated double-stranded RNA molecule capable of target-specific RNA interference, having 3 to 10 G/C base pairs added to the target sequence at the 5' end of the passenger strand and the 3' end of the guide strand such that said RNA molecule has an increased thermodynamic stability at the 5' end of the passenger strand and the 3' end of the guide strand, respectively, in comparison to the base paring of the first 10 nucleotides at the 5' end of the guide strand and wherein 3 to 10 G nucleotides are added to the 5' end of the target sequence at the passenger strand and 3 to 10 C nucleotides are added to the 3' end of the target sequence at the guide strand, wherein the 3' end of the passenger strand has no overhang or has an overhang of 1 to 3 nucleotides , wherein the RNA molecule has a length of 19 to 22 nucleotides excluding the G/C base pair sequences at the 5' end of the passenger strand and the 3' end of the guide strand, respectively, and excluding the optional overhang at the 3' end of the passenger strand, if present.

2. The RNA molecule of claim 1 wherein the overhang at the 3' end of the passenger strand is linked to a fluorophore or chromophore.

3. The RNA molecule of claim 1 or 2 wherein the terminal nucleotide at the 5' end of the passenger strand has a monophosphate group at the 5' position of the ribose.

4. The RNA molecule of claim 1 or 2 wherein the terminal nucleotide at the 5' end of the passenger strand has a triphosphate group at the 5' position of the ribose.

5. The RNA molecule according to any one of claims 1 to 4 for use as a medicament.

6. A pharmaceutical composition comprising the RNA molecule according to any one of claims 1 to 4 in combination with at least one pharmaceutical carrier, excipient and/or diluent.

7. Eukaryotic cell or eukaryotic non-human organism comprising the RNA molecule according to any one of claims 1 to 4 or a DNA molecule coding therefor.

## Patentansprüche

1. Isoliertes doppelsträngiges, zur zielspezifischen RNA-Interferenz befähigtes RNA-Molekül, das 3 bis 10 G/C-Paare, die an die Zielsequenz am 5'-Ende des Passenger-Strangs und am 3'-Ende des Guide-Strangs angefügt sind, derart aufweist, dass die thermodynamische Stabilität am 5'-Ende des Passenger-Strangs bzw. am 3'-Ende des Guide-Strangs im Vergleich zur Basenpaarung der ersten 10 Nukleotide am 5'-Ende des Guide-Strangs erhöht ist, und wobei 3 bis 10 G-Nukleotide am 5'-Ende der Zielsequenz am Passenger-Strang angefügt sind und 3 bis 10 C-Nukleotide am 3'-Ende der Zielsequenz am Guide-Strang angefügt sind, wobei das 3'-Ende des Passenger-Strangs keinen Überhang oder einen Überhang von 1 bis 3 Nukleotiden aufweist, wobei das RNA-Molekül eine Länge von 19 bis 22 Nukleotiden, ausgenommen die G/C-Basenpaare am 5'-Ende des Passenger-Strangs bzw. am 3'-Ende des Guide-Strangs und, sofern vorhanden, ausgenommen den gegebenenfalls vorhandenen Überhang am 3'-Ende des Passenger-Strangs, aufweist.

2. RNA-Molekül nach Anspruch 1, wobei der Überhang am 3'-Ende des Passenger-Strangs mit einem Fluorophor oder Chromophor verbunden ist.

3. RNA-Molekül nach Anspruch 1 oder 2, wobei das terminale Nukleotid am 5'-Ende des Passenger-Strangs eine Monophosphatgruppe in der 5'-Position der Ribose aufweist.

4. RNA-Molekül nach Anspruch 1 oder 2, wobei das terminale Nukleotid am 5'-Ende des Passenger-Strangs eine Triphosphatgruppe in der 5`-Position der Ribose aufweist.

5. RNA-Molekül nach einem der Ansprüche 1 bis 4 zur Verwendung als Medikament.

6. Pharmazeutische Zusammensetzung, die das RNA-Molekül nach einem der Ansprüche 1 bis 4 in Kombination mit mindestens einem pharmazeutisch verträglichen Träger, Vehikel und/oder Verdünnungsmittel umfasst.

7. Eukaryontische Zelle oder eukaryontischer nicht-humaner Organismus, die bzw. der das RNA-Molekül nach einem der Ansprüche 1 bis 4 oder eine dafür codierende DNA umfasst.

## Revendications

1. Molécule d'ARN isolé à double brin capable d'une interférence d'ARN spécifique à la cible, ayant de 3 à 10 paires de base G/C ajoutées à la séquence cible à l'extrémité 5' du brin passager et de l'extrémité 3' du brin guide de telle sorte que ladite molécule d'ARN présente une stabilité thermodynamique augmentée à l'extrémité 5' du brin passager et de l'extrémité 3' du brin guide, respectivement, en comparaison à l'appariement de base des 10 premiers nucléotides à l'extrémité 5' du brin guide et dans laquelle de 3 à 10 nucléotides G sont ajoutés à l'extrémité 5' de la séquence cible au niveau du brin passager et de 3 à 10 nucléotides C sont ajoutés à l'extrémité 3' de la séquence cible au niveau du brin guide, dans laquelle l'extrémité 3' du brin passager n'a pas de dépassement ou a un dépassement de 1 à 3 nucléotides, dans laquelle la molécule d'ARN a une longueur de 19 à 22 nucléotides à l'exclusion des séquences de paires de base G/C à l'extrémité 5' du brin passager et à l'extrémité 3' du brin guide, respectivement, et à l'exclusion du dépassement optionnel à l'extrémité 3' du brin passager, le cas échéant.

2. Molécule d'ARN selon la revendication 1, dans laquelle le dépassement à l'extrémité 3' du brin passager est lié à un fluorophore ou chromophore.

3. Molécule d'ARN selon la revendication 1 ou 2, dans laquelle le nucléotide terminal à l'extrémité 5' du brin passager comporte un groupe monophosphate dans la position 5' du ribose.

4. Molécule d'ARN selon la revendication 1 ou 2, dans laquelle le nucléotide terminal à l'extrémité 5' du brin passager comporte un groupe triphosphate dans la position 5' du ribose.

5. Molécule d'ARN selon l'une quelconque des revendications 1 à 4 pour utilisation en tant que médicament.

6. Composition pharmaceutique comprenant la molécule d'ARN selon l'une quelconque des revendications 1 à 4 en combinaison avec au moins un vecteur, un excipient et/ou un diluant pharmaceutique.

7. Cellule eucaryote ou organisme non humain eucaryote comprenant la molécule d'ARN selon l'une quelconque des revendications 1 à 4 ou une molécule d'ADN codant pour celui-ci.


### SEQUENCE LISTING

<110> Riboxx GmbH
<120> Improved design of small-interfering RNA
<130> R 0061 WO
<150> EP 09153487.5
   <151> 2009-02-24
<160> 26
<170> Patent In version 3.3
<210> 1
   <211> 21
   <212> DNA
   <213> Artificial
<220>
   <223> T-siRNA sense strand
<400> 1
   acucugagcu ucgugcuuat t 21
<210> 2
   <211> 21
   <212> DNA
   <213> Artificial
<220>
   <223> T-siRNA antisense strand
<400> 2
   uaagcacgaa gcucagagut t 21
<210> 3
   <211> 19
   <212> RNA
   <213> Artificial
<220>
   <223> blunt-siRNA sense strand
<400> 3
   acucugagcu ucgugcuua 19
<210> 4
   <211> 19
   <212> RNA
   <213> Artificial
<220>
   <223> blunt-siRNA antisense strand
<400> 4
   uaagcacgaa gcucagagu 19
<210> 5
   <211> 24
   <212> RNA
   <213> Artificial
<220>
   <223> RiboxX-siRNA sense strand
<400> 5
   gggggacucu gagcuucgug cuua 24
<210> 6
   <211> 24
   <212> RNA
   <213> Artificial
<220>
   <223> RiboxX-siRNA antisense strand
<400> 6
   uaagcacgaa gcucagaguc cccc 24
<210> 7
   <211> 21
   <212> RNA
   <213> Artificial
<220>
   <223> T-siRNA-1 sense strand
<400> 7
   gcuuguggcu auucagauau u 21
<210> 8
   <211> 21
   <212> RNA
   <213> Artificial
<220>
   <223> T-siRNA-1 antisense strand
<400> 8
   uaucugaaua gccacaagcu u 21
<210> 9
   <211> 21
   <212> RNA
   <213> Artificial
<220>
   <223> T-siRNA 2 sense strand
<400> 9
   ugaaagugau guguauucau u 21
<210> 10
   <211> 21
   <212> RNA
   <213> Artificial
<220>
   <223> T-siRNA-2 antisense strand
<400> 10
   ugaauacaca ucacuuucau u 21
<210> 11
   <211> 21
   <212> RNA
   <213> Artificial
<220>
   <223> T-siRNA 3 sense strand
<400> 11
   uuggucagau ucuagguauu u 21
<210> 12
   <211> 21
   <212> RNA
   <213> Artificial
<220>
   <223> T-siRNA-3 antisense strand
<400> 12
   auaccuagaa ucugaccaau u 21
<210> 13
   <211> 21
   <212> RNA
   <213> Artificial
<220>
   <223> T-siRNA-4 sense strand
<400> 13
   gcaaguagca uaaaugauau u 21
<210> 14
   <211> 21
   <212> RNA
   <213> Artificial
<220>
   <223> T-siRNA-4 antisense strand
<400> 14
   uaucauuuau gcuacuugcu u 21
<210> 15
   <211> 21
   <212> RNA
   <213> Artificial
<220>
   <223> T-siRNA-5 sense strand
<400> 15
   acugcugaca uguuguguuu u 21
<210> 16
   <211> 21
   <212> RNA
   <213> Artificial
<220>
   <223> T-siRNA-5 antisense strand
<400> 16
   aacacaacau gucagcaguu u 21
<210> 17
   <211> 23
   <212> RNA
   <213> Artificial
<220>
   <223> Riboxx-siRNA-1 sense strand
<400> 17
   gggggcuugu ggcuauucag aua 23
<210> 18
   <211> 23
   <212> RNA
   <213> Artificial
<220>
   <223> Riboxx-siRNA-1 antisense strand
<400> 18
   uaucugaaua gccacaagcc ccc 23
<210> 19
   <211> 24
   <212> RNA
   <213> Artificial
<220>
   <223> Riboxx-siRNA-2 sense strand
<400> 19
   gggggugaaa gugaugugua uuca 24
<210> 20
   <211> 24
   <212> RNA
   <213> Artificial
<220>
   <223> Riboxx-siRNA-2 antisense strand
<400> 20
   ugaauacaca ucacuuucac cccc 24
<210> 21
   <211> 24
   <212> RNA
   <213> Artificial
<220>
   <223> Riboxx-siRNA-3 sense strand
<400> 21
   ggggguuggu cagauucuag guau 24
<210> 22
   <211> 24
   <212> RNA
   <213> Artificial
<220>
   <223> Riboxx-siRNA-3 antisense strand
<400> 22
   auaccuagaa ucugaccaac cccc 24
<210> 23
   <211> 23
   <212> RNA
   <213> Artificial
<220>
   <223> Riboxx-siRNA-4 sense strand
<400> 23
   gggggcaagu agcauaaaug aua 23
<210> 24
   <211> 23
   <212> RNA
   <213> Artificial
<220>
   <223> Riboxx-siRNA-4 antisense strand
<400> 24
   uaucauuuau gcuacuugcc ccc 23
<210> 25
   <211> 24
   <212> RNA
   <213> Artificial
<220>
   <223> Riboxx-siRNA-5 sense strand
<400> 25
   gggggacugc ugacauguug uguu 24
<210> 26
   <211> 24
   <212> RNA
   <213> Artificial
<220>
   <223> Riboxx-siRNA-5 antisense strand
<400> 26
   aacacaacau gucagcaguc cccc 24